# EUROPEAN PATENT APPLICATION

(11) **EP 3 477 304 A1**
(43) Date of publication of application: **01.05.2019**
(21) Application number: 17839266.8
(22) Date of filing: 31.07.2017
(51) Int. Cl.: G01N 33/574, G01N 33/48, G01N 33/53, G01N 33/536

(54) **METHOD RELATING TO EVALUATION OF TUMOR TISSUE OF EXPERIMENTAL ANIMAL**

(30) Priority: 08.08.2016 JP 2016155727
(71) Applicant: Konica Minolta, Inc., Tokyo 100-7015 (JP)
(72) Inventor: KURIHARA, Yoshikazu, Tokyo 100-7015 (JP); GOUDA, Hideki, Tokyo 100-7015 (JP); ISODA, Takeshi, Tokyo 100-7015 (JP); SHIRAISHI, Takeshi, Tokyo 100-7015 (JP)
(74) Representative: Gille Hrabal
(86) International application number: PCT/JP2017/027648
(87) International publication number: WO 2018/030193

(57) **Abstract**

The present invention relates to a method relating the evaluation of tumor tissue from an experimental animal, the method including the step of measuring an amount of a substance, except for human-derived tumor cells, contained in a sample collected from a region in an experimental animal into which a human-derived tumor tissue ortumor cells have been transplanted, wherein the region contains the tumor tissue or the tumor cells.

## Description

### Technological Field

The present invention relates to a method that is applied to an experimental animal such as a PDX (patient-derived tumor xenograft) model. In particular, the present invention relates to a method that can be used to evaluate tumor tissue from such an experimental animal.

### Background

In studies on cancers (tumors), animal models produced by transplanting a human-derived tumor tissue or tumor cells into immunodeficient experimental animals, such as tumor-bearing mice produced by using mouse as such an experimental animal, may be used as an experimental system that recaptures the *in vivo* environment.

There are roughly two known types of tumor-bearing mice. One of them refers to cultured cell-introduced mouse model, which is established by implanting cultured cells derived from tumor cells extracted from a patient into a mouse, and incubating those cells in the mouse. The other refers to tumor tissue-transplanted mouse model, which is established by implanting a tumor tissue or tumor cells extracted from a patient into a mouse and incubating the tissue or the cells in the mouse to produce such a mouse model. Such tumor-bearing mice can be used to assess the efficacy and the safety (toxicity) of a drug or a candidate compound for the drug in research and development process or the nonclinical stage, which is prior to any test on human subjects. Moreover, the feasibility to produce mice carrying tumors derived from patients with cancers of various properties enables comparative analyses using such mice to find out the profiles of sensitive patients.

The former type of tumor-bearing mice can be easily produced and thus have been classically established as experimental animals. Cells used for the production of such mice are cloned by culturing *in vitro* and the cloned cells are transplanted into mice to make the resulting mice stably maintain those clonal components and these mice are thus convenient for studies that require use of tumor-bearing mice with small individual variations. However, even use of such tumor-bearing mice may often result in failure to prepare homogeneous sample slides. Thus, the commercialization of sample slides prepared using such mice need to conduct a quality assurance program by use of an internal control or implementation of a pre-shipment inspection program.

On the other hand, the latter type of tumor tissue-transplanted mouse models require a less reliable procedure to be performed, in which a tumor tissue or tumor cells extracted from a patient are directly transplanted into mice, and such mouse models have been so far produced in a limited number of facilities. However, the fact that the complexity of tumor cells including the cause of generating the complexity of the tumor cells (such as genetic mutations) are maintained and reproduced over generations has recently led to the recognition of the importance of studies using such tumor-bearing mice.

A PDX mouse model is given as an example of tumor-bearing mouse that potentially maintains and reproduces the above-described complexity at or above a certain level. PDX is short for patient-derived tumor xenograft and refers to implantation of a patient- (human-)derived tumor tissue or tumor cells in a mouse or another experimental animal and subsequent incubation of the implant for a certain period of time in the mouse or said another animal. PDX model mice include mouse individuals into which a tumor tissue or tumor cells removed from a patient have been transplanted for the first time (the primary mouse in the 0-th generation) and mouse individuals into which the tumor tissue or the tumor cells (the tumor region) grown in the body of the primary mouse have been transplanted (the passage mice, for example, in the first and the second generations).

Cells cultured *in vitro* are not used in PDX model mice produced in this way. Therefore, it is believed that use of such a PDX mouse model enables evaluation of efficacy and safety by better reproducing the actual human pathological conditions than use of a conventional tumor-bearing mouse. Diagnostic and therapeutic methods (methods for getting indices useful in diagnosis or treatment) based on non-clinical test methods using PDX model mice are increasingly developed in recent years. PDX models can be produced based not only on mice but also on various experimental animals. One can refer to, for example, Non-Patent Document 1 for the PDX model mice and other various tumor-bearing mice.

It is increasingly understood that the properties of (namely, the expressed genes and expressed proteins) a tumor tissue or tumor cells contained in a tumor region are not always completely maintained through passages but are altered even in PDX experimental animal models. For example, Non-Patent Document 2 describes the establishment of PDX mouse models of bladder cancer and illustrates various mice showing properties depending on patients or type of proteins, such as mice showing properties that have been significantly altered, have not been altered, and have been gradually altered through passages, from those of the original patient tumor sample (see, for example, Figure 3). In other words, PDX experimental animal models inherit genetic information through passages but are not as clonally homogeneous as cultured cell-introduced models. Thus, sample slides prepared from those tumor-bearing mice may not be same. Therefore, quality assurance tests are seen as more important for sample slides prepared by using PDX experimental animal models than for those prepared by using cultured cell-introduced models.

### Related Art Documents

### Non Patent Documents

Non-Patent Document 1: Sanmamed et al., Defining the optimal murine models to investigate immune checkpoint blockers and their combination with other immunotherapies. Annals of Oncology 00: 1-9, 2016.
Non-Patent Document 2: Jager et al., Patient-derived bladder cancer xenografts in the preclinical development of novel target therapies. Oncotarget, Vol.6, No. 25, 21522-21532.

### Summary

### Technical Problem

Even in experimental animals such as a PDX mouse model, the properties of tumor cells are not always completely maintained between primary and passaged PDX mouse models, as described above, and PDX mouse models currently lack sufficient quality control. When such a PDX mouse model is used to conduct research on, for example, drug efficacy, it is not clear whether or not the mouse model successfully reproduces the result of actual administration of the drug to a patient, and the analysis may thus end in failure. This is suspected to be a reason why the popularity of PDX mouse model is lower than expected at first.

An embodiment of the present invention provides a means to perform quality control on experimental animals into which a patient- (human-)derived tumor tissue or tumor cells have been transplanted, such as PDX mouse models, or to analyze the results of tests using such experimental animals, in a more precise manner.

### Solution to Problem

The inventor assumed that one of the causes of the above-described problem was changes in tumor properties from the original due to the infiltration of, for example, fibroblast, extracellular matrix, endothelial cells, lymphocyte, monocyte, and other leukocyte all derived from an experimental animal into a region (a tumor region) containing a patient-derived tumor tissue or tumor cells. Then, the inventor found that quantification of substance, except for human-derived tumor cells, in a tumor tissue-derived sample collected from a tumor region of an experimental animals, such as stromal cell from an experimental animal as described above, detected changes in properties of the tumor tissue, which was a problem associated with the use of the experimental animal, and enabled laboratory animal quality control and/or more quantitative tumor analysis.

In other words, the present invention provides, in one aspect, a method including the step of measuring an amount of a substance, except for human-derived tumor cells, contained in a sample collected from a region in an experimental animal into which a human-derived tumor tissue or tumor cells have been transplanted, wherein the region contains the tumor tissue or the tumor cells.

### Advantageous Effects of Invention

The amount of a substance except for human-derived tumor cells, such as stromal cell from an experimental animal, is measured by a method according to one embodiment of the present invention. When the resulting amount is relatively large (for example, larger than a predetermined reference value), the tumor tissue maintained by the experimental animal from which the sample has been collected may potentially have relatively large changes in properties as compared to the tumor tissue in an original patient or primary experimental animal, and the effectivity of agents such as anti-cancer drugs in the experimental animal and in experimental animals in subsequent passage may potentially be different from that in the primary experimental animal. Use of the method according to the present invention by which the above-described analysis can be performed enables quality control of various experimental animals, such as a PDX mouse model, and analysis of the results of tests using such experimental animals with a higher level of accuracy than ever before. Thus, it can be expected that nonclinical studies using such experimental animals can be actively utilized in various settings.

### Brief Description of the Drawings

Fig. 1 is a schematic diagram for Example 1.
Fig. 2 is a schematic diagram for Example 2.

### Description of Embodiments

A method according to one embodiment of the present invention includes the step of measuring the amount of a substance, except for human-derived tumor cells, contained in a sample collected from a region (tumor region) in an experimental animal into which a human-derived tumor tissue or human-derived tumor cells have been transplanted, wherein the region contains the tumor tissue or the tumor cells.

The term "tumor" in the present invention usually refers to malignant tumor and includes, for example, "cancer (meaning cancer or carcinoma in English)," which means malignant tumors arise in epithelial cells of, for example, the skin, the stomach mucosa and the intestinal mucosa; "sarcoma (meaning sarcoma in English)," which means malignant tumors arise in non-epithelial cells, such as muscle cells, fibrous cells, bone cells, fat cells, vascular cells, and nerve cells; and leukemia and malignant lymphoma that arise in the hematopoietic organs. In this specification, the term "tumor tissue" or "tumor cell" is commonly used for explanation purposes and, in typical embodiments, the term "cancer tissue" or "cancer cell" may also be used for explanation purposes.

The "experimental animal" used in the present invention is an experimental animal into which a human-derived tumor tissue or human-derived tumor cells have been transplanted, namely an experimental animal commonly referred to as a tumor-bearing animal. Immunodeficient animals are usually used for the production of PDX models to inhibit rejection of a transplanted human-derived tumor tissue or tumor cells. Examples of the animal include animals which have been genetically controlled to some extent and have homogeneous genetic backgrounds, such as mouse, rat, rabbit, guinea pig, gerbil, hamster, ferret, dog, miniature pig, monkey, cow, horse, and sheep.

For an experimental animal in the 0-th generation, the "human-derived tumor tissue or tumor cells" to be transplanted into an experimental animal refer to a tumor tissue or tumor cells that have been collected from a human individual (patient) or to human-derived tumor cells of an established cultured cell line and, for an experimental animal in the n-th generation (n ≥ 1), refer to a tumor tissue or tumor cells that are originated from the tumor tissue or the tumor cells transplanted in such an experimental animal in the 0-th generation and have grown in an experimental animal in the (n-1)-th generation. The method according to one embodiment of the present invention is preferably applied for the purpose of, for example, quality improvement to an experimental animal (such as a PDX mouse model) in the n-th generation that shows relatively large changes in the properties of a tumor tissue or tumor cells contained in a tumor region, which have first been collected from a human individual (patient) and transplanted into an experimental animal in the 0-th generation and then serially passaged. The method may also be applied for the purpose of, for example, further quality improvement to an experimental animal (such as a cultured cell-introduced mouse model) in the n-th generation that shows relatively small changes in the properties of a tumor tissue or tumor cells, which have first been transplanted as human-derived tumor cells of an established cultured cell line into an experimental animal in the 0-th generation and then serially passaged.

The location in an experimental animal into which a human-derived tumor tissue or tumor cells are transplanted is not particularly limited to, for example, subcutaneous locations, intradermal locations, organs, and intracranial locations, but subcutaneous transplantation in the back or the flank is preferred in terms of operational convenience and/or easy sample collection if the experimental animal is, for example, mouse.

The procedure of transplanting a human-derived tumor tissue or tumor cells into an experimental animal to produce a tumor-bearing animal is not limited to a particular procedure, and any known procedure can be used. In cases where, for example, the experimental animal is mouse, a procedure of producing a tumor-bearing mouse model by means of PDX is preferably used and the environment that allows purchase of any finalized tumor-bearing mouse model is well established. On the other hand, tumor-bearing mouse models transplanted with cultured cells that are derived from tumor cells extracted from a patient can be produced by any known procedure and are also obtainable from various institutions.

CDX (cell-line derived xenograft) mouse model is given as an example of a mouse into which cultured cancer cells extracted from a patient have been transplanted, while examples of a mouse into which a human-derived tumor tissue or tumor cells have been transplanted include, for example, Immuno-Avatar mouse models, Hemato-Lymphoid humanized mouse models, and Immune-PDX mouse models, in addition to PDX (patient-derived xenograft) mouse models (Sanmamed et al., Annals of Oncology 00: 1-9, 2016).

The "tumor region" in every experimental animal in each generation refers to a region containing "a human-derived tumor tissue or tumor cells" as described above, from which a "sample" is collected. The tumor region contains not only a human-derived tumor tissue or tumor cells but also "substances except for the human-derived tumor cells". In the present invention, the amount of "a substance except for human-derived tumor cells" contained in a sample collected from the tumor region is measured. One or more measurement objects may be included in the substance except for human-derived tumor cells.

In this specification, a "substance except for human-derived tumor cells," which is always quantified in the present invention, is sometimes referred to as a "first target substance," while a "substance as a drug efficacy evaluation object owned by the human-derived tumor cells," which is further quantified in a preferred embodiment of the present invention, is sometimes referred to as a "second target substance".

In this specification, the "sample" is prepared from a tumor region in an experimental animal, as described above. The method of collecting a tumor region from an experimental animal is not limited to a particular method, but a surgical knife may be used to remove the entire part of the tumor region or a biopsy needle may be used to collect a part of the tumor region. A biopsy needle is preferably used for collection purposes because an experimental animal into which a biopsy needle has been inserted to collect a part of a tumor can be later used in efficacy tests, for example, for anti-cancer drugs.

The "sample" collected from the tumor region preferably takes the form of a sample slide that has been prepared according to a predetermined procedure, such as those conventionally used, for example, for determining the expression level of a target protein by immunostaining, for example, in pathological diagnosis. The method according to one embodiment of the present invention is performed on an experimental animal by using such a sample *ex vivo.*

The "substance except for human-derived tumor cells" contained in a tumor region or in a sample collected therefrom is typically "a stroma derived from an experimental animal" or "a stroma derived from a human-derived tumor tissue". A stroma derived from an experimental animal is a tissue derived from the experimental animal and infiltrated into the tumor region in the body of the same experimental animal. On the other hand, a stroma derived from a human-derived tumor tissue in a mouse into which the human-derived tumor tissue or tumor cells have been transplanted is a stroma contained in the human-derived tumor tissue, which has first been collected from a human individual (patient) and then transplanted into an experimental animal in the 0-th generation, or a tissue derived from the stroma. An increase in the ratio of the stroma derived from the experimental animal to the tumor tissue or the sample relatively reduces the ratio of the stroma derived from the human-derived tumor tissue to the tumor tissue or the sample.

The "stroma" is mainly made of stromal cells such as fibroblast, endothelial cell, and leukocyte (lymphocyte, monocyte, neutrophil, eosinophil, and basophil), and extracellular matrix made mainly from proteins such as collagen and proteoglycan. In one embodiment of the present invention, either of the stromal cells and the extracellular matrix may be quantified, but the quantification of stromal cells, which are considered to have much more effect on the properties of human-derived tumor tissue carried by the experimental animal, is preferred. Moreover, the quantification of fibroblast, which is a representative stromal cell, is preferred.

In cases where stromal cell is selected as a substance except for human-derived tumor cells, the method of quantifying the stromal cell is not limited to a particular method, but various known methods can be used. A stromal cell marker or the like, that is, a marker protein that is expressed on the stromal cell surface (cell membrane) and can distinguish stromal cells from other types of cells is preferably used to quantify stromal cells by immunostaining using an antibody against the marker protein. Moreover, for example, any protein other than the marker protein, nucleic acids related to the marker protein or the other protein (such as mRNAs), and sugars that modify the protein may be used to stain and quantify stromal cells, as long as stromal cells can be distinguished from other types of cells, at least from human-derived tumor cells.

In one embodiment of the present invention, for example, a stromal cell marker as described above itself may be considered as a "substance except for human-derived tumor cells". A stromal cell marker or the like is a substance that reflects the quantity of stromal cells and an increase in the quantity of stromal cells almost proportionally increases the quantity of the stromal cell marker. Thus, the quantity of the stromal cell marker or the like may be measured as an alternative to the quantity of stromal cells.

As a stromal cell marker, an appropriate membrane protein selected from, for example, those as indicated below can be used. As one example, CD140a is a membrane protein expressed on the cell surface, such as that of fibroblast, megakaryocyte, monocyte, erythrocyte, bone marrow precursor cells, and endothelial cells, and is preferable as a stromal cell marker in one embodiment of the present invention.
CD106 (VCAM-1, INCAM-110; CD49d/CD29-L): activated vascular endothelial cells and dendritic cells (DCs);
CD109 (Platelet activation factor, 8A3, E123): activated T cells, platelet, vascular endothelial cells, megakaryocyte, and a CD34+ subset of progenitor cells;
CD140a (PDGF-R, PDGFR2): fibroblast, megakaryocyte, monocyte, erythrocyte, bone marrow precursor cells, and endothelial cells;
CD140b (PDGF-R, PDGFR1): endothelial cells and stromal cells;
CD141 (Thrombomodulin): vascular endothelial cells, bone marrow cells, platelet, and smooth muscle ;
CD142 (Tissue Factor (TF), Thromboplastin): epithelial cells, activated monocyte, and activated vascular endotherium;
CD143 (ACE; Angiotensin-converting enzyme): vascular endotherium, epithelial cells, and activated macrophages;
CD144 (VE-Cadherin, Cadherin-5: related to vascular endothelial permeability): vascular endotherium;
CD145 (7E9, P7A5): a marker of endothelial cells;
CD146 (MUC18, s-endo, Mel-CAM): vascular endotherium, activated T cells, and melanoma;
CD147 (Basigin, M6, EMMRRIN): leukocytes, erythrocyte, vascular endotherium, and platelet;
CD201 (EPCR: protein C receptor): vascular endotherium;
CD202 (TIE2, TEK; Angiopoietin-1-R): vascular endotherium and a subset of hematopoietic stem cells;
CD280 (Endo180, TEM22, uPARAP (uPAR-associated protein)): myeloid precursor cells, fibroblast, a subset of endothelial cells, and a subset of macrophages;
CD299 (DC-SIGN-related, L-SIGN (Liver/Lympho node specific ICAM3-grabbing nonintegrin)): endothelial cells;
CD309 (VEGFR2: Vascular endothelial growth factor receptor 2, KDR): endothelial cells, megakaryocyte, platelet, and a subset of stem cells;
CD322 (JAM2: Junctional adhesion molecule 2): endothelial cells, monocyte, B cells, and a subset of T cells;
CD331 (FGFR1: Fibroblast growth factor receptor 1): fibroblast and epithelial cells;
CD332 (FGFR2, Keratinocyte growth factor receptor): epithelial cells;
CD333 (FGFR3, JTK4; Achondroplasia, Thanatophoric dwarfism): fibroblast and epithelial cells;
CD334 (FGFR4, JTK2, TKF): fibroblast and epithelial cells;
CD339 (Jagged-1, JAG1; Alagille syndrome): stromal cells and epithelial cells.

The amount of a substance, except for human-derived tumor cells, in a sample is generally measured by observation and imaging of a sample slide under dark field or bright field and is usually obtained as the amount of the substance contained in a region corresponding to one image frame or the average amount of the substance contained in regions corresponding to multiple image frames.

In the method according to one embodiment of the present invention, an identical sample slide may be used to quantify a first target substance (and a second target substance) and also to obtain, as necessary, further information such as the types, numbers, morphologies, and manifestation locations (distribution and occupation area in a tumor tissue or a sample) of cells that contain the first target substance (and the second target substance).

Moreover, combination of the quantification result and the further information as described above in terms of the first target substance and/or the second target substance draws out new information on one or more topics, which can be used for desired purposes. Examples of such new information include information on, for example, (i) the (average) amount(s) of the first target substance and/or the second target substance per one cell, (ii) the amount(s) of the first target substance and/or the second target substance per unit tissue area, (iii) a histogram showing the number of cells corresponding to each of the respective amounts of the first target substance and/or the second target substance per one cell, and (iv) a curve showing the number of cells corresponding to each of the respective expression levels of the first target substance and/or the second target substance per cell, in each sample (sample slide). The methods of obtaining information on each of the above-described topics will be described below in accordance with an embodiment in which the expression level of a protein selected as the first target substance or the second target substance (hereinafter referred to as a "protein of interest") is measured by immunostaining using fluorescent nanoparticles.
(i) In cases where the average expression level of a target protein per one cell is quantified, a sample (sample slide) is, for example, immunostained with fluorescent nanoparticles and simultaneously stained with a staining agent for morphological observation (for example, eosin) to identify cellular morphology. An image showing the target protein labeled by the fluorescent nanoparticle and viewed as a bright spot is obtained by observation and imaging under dark field along with applying excitation light with a defined wavelength relevant to the fluorescent nanoparticles. On the other hand, an image of cells that have been stained to show their morphology is obtained by observation and imaging under bright field. These two images are processed to create an overlay image, whereby the number of bright spots each representing the expressed target protein can be counted in individual cells included in the entire area of the image or in a particular region of the image (for example, only in a tumor tissue). The number of counted bright spots may be considered as an index for the expression level of the target protein. Moreover, multiple fluorescent nanoparticles may form one bright spot and, thus, the brightness (luminacce, fluorescence intensity) of one bright spot at a certain location can be divided by the separately measured brightness of one fluorescent nanoparticle to calculate the number of fluorescent nanoparticles constituting the bright spot, and the resulting particle number may be used as an index for the expression level of the target protein. The average expression level per cell can be quantified by counting the number of bright spots or fluorescent particles in all cells included in the image.
(ii) In cases where the expression level of the target protein per unit tissue area is quantified, the total number of bright spots or fluorescent particles in a particular tissue region included in the image should be counted similarly to the method (i) and then divided by the tissue area.
(iii)In cases where a histogram showing the number of cells corresponding to each of the respective expression levels of the target protein per one cell is generated, the number of bright spots or fluorescent particles each representing the expressed target protein is first counted similarly to the method (i) in individual cells included in the entire area of the image or in a particular region of the image (for example, only in a tumor tissue). A histogram can then be generated by plotting a series of equally spaced bins, each representing a range of expression levels of the target protein per cell, on the horizontal axis and the measured number of cells (frequency) corresponding to each of the respective bins on the vertical axis.
(iv)In cases where a curve showing the number of cells corresponding to each of the respective expression levels of the target protein per one cell is generated, the number of bright spots or fluorescent particles each representing the expressed target protein is first counted similarly to the method (i) in individual cells included in the entire area of the image or in a particular region of the image (for example, only in a tumor tissue). A curve can then be generated by plotting the expression level of the target protein per cell in a continuous fashion (without being divided as in the case of the histogram) on the horizontal axis and the measured number of cells (frequency) corresponding to each of the respective expression levels on the vertical axis.

The histogram obtained by the above-described method (iii) and the curve obtained by the above-described method (iv) may give information, for example, on the state of distribution (the shape of the histogram or curve, and the number of peaks), mean value or median and the variance value (the value of CV), and also, particularly in the case of the histogram, the number of cells corresponding to the bin which represents the largest number of bright spots or fluorescent particles per one cell (frequency).

The quantification method for a substance except for human-derived tumor cells (a first target substance) is not limited to a particular method as long as the first target substance can be quantified at a desired accuracy level. In cases where, for example, a type of stromal cell is selected as a first target substance, the type of stromal cells can be quantified as described above by immunostaining using a stromal cell marker. According to one embodiment of the present invention, embodiments using two representative quantification methods as described below are preferred.

The first embodiment of quantification method (the first quantification method) is a technique to label and quantify a first target substance (for example, the number of stromal cells or the number of stromal cell markers almost proportional thereto) by using particles with a diameter in nanometer scale, such as fluorescent nanoparticle, *i.e.,* quantum dot (not integrated), or phosphor integrated dot (PID), which is an assembly of a matrix such as resin and a fluorophore such as fluorescent dye or quantum dot. Among those, a quantification method using PID as disclosed in Examples below (referred to herein as "PID method") is highly sensitive to detect a first target substance and is available for precise quantification and is therefore particularly preferable. In cases where, for example, a stromal cell marker expressed in a type of stromal cell or on the cell membrane of the stromal cell is selected as a first target substance, the amount of the first target substance has a positive correlation with the number of bright spots generated by fluorescent nanoparticles, preferably by PIDs, or with the number of particles bound to the first target substance (the brightness of one bright spot can be divided by the brightness of one fluorescent nanoparticle to calculate the number of particles constituting the bright spot), and the number of such bright spots or fluorescent particles tends to increase as the amount of the first target substance increases.

Basic embodiments for protein quantification using the PID method have been already disclosed in WO2012/029752, WO2013/035703, or other patent or non-patent documents. Also in one embodiment of the present invention, the PID method can be implemented, for example, by one embodiment in accordance with an embodiment in which sample slides are used for pathological diagnosis.

The second embodiment of quantification method (the second quantification method) is a staining technique commonly used in the conventional IHC (immunohistochemistry) method, in which the enzyme-substrate reaction is used. Among those, the DAB staining method, in which the reaction of a peroxidase with diaminobenzidine (DAB) is used, has an excellent staining ability and is therefore particularly preferable. In use of the DAB staining method, a first target substance is labeled with an enzyme (peroxidase) and then allowed to react with its substrate, diaminobenzidine (DAB), for chromogenic reaction, which consequently stains the first target substance and its surrounding region in brown. In cases where, for example, a stromal cell marker expressed in a type of stromal cell or on the cell membrane of the stromal cell is selected as a first target substance, the amount of the first target substance has a positive correlation with the amount of the pigment generated from DAB, *i.e.,* the number of cells stained in brown or the area ratio of the region stained in brown to a field of view on a sample slide, or with the intensity of the brown color, and the number of such cells or the area ratio tends to increase or, alternatively, the color tends to darken to dark brown as the amount of the first target substance increases.

The indices obtained by the first quantification method and the second quantification method, *i.e.,* for example, the number of particles such as phosphor integrated dots (PIDs) obtained by the first quantification method and the area ratio of a region stained by a pigment generated by an enzyme reaction to a field of view on a sample slide obtained by the second quantification method, reflect the amount of the substance except for human-derived tumor cells. Moreover, such index values may be stratified (scored) into several (for example, 2 to 5) predefined ranges of values. In other words, a set of samples with index values within a predetermined range may be placed in the same group.

Moreover, the amount of the first target substance may be measured by the first quantification method using a fluorescent label such as fluorescent nanoparticle, preferably PID, or by the second quantification method using an enzyme-substrate reaction, such as DAB staining, and is preferably measured by the former method, which has a more excellent quantifying ability and enables drug efficacy evaluation with high accuracy.

In a preferred embodiment, the method according to one embodiment of the present invention further includes the step of measuring the amount of a substance as a drug efficacy evaluation object owned by the human-derived tumor cells.

The "substance as a drug efficacy evaluation object owned by the human-derived tumor cells," that is, the second target substance in one embodiment of the present invention is not limited to a particular substance but can be selected from various known substances. One or more measurement objects may be included in the second target substance.

Examples of the second target substance include proteins expressed in tumor cells and targeted by molecularly targeted drugs, such as, particularly, EGFR (HER1), HER2, HER3, VEGFR, PD-L1, Bcr-Abr, Kit, ALK, JAK, Btk, PI3K, BRAF, mTOR, and CDK4/6.

Moreover, examples of the second target substance also include nucleic acids related to cell membrane proteins in tumor cells as described above, such as a mRNA coding for the membrane protein or a gene that may be amplified in tumor cells, such as *HER2,* and sugars (sugar chains) that modify cell membrane proteins in tumor cells, such as a sugar (sugar chain) which is different from the sugar moiety of a cell membrane protein in normal cells as a consequence of neoplastic transformation.

The amount of the second target substance may be measured by the same methods as the above-described methods for the first target substance. In cases where, for example, a protein is selected as a second target substance, the amount of the protein is preferably measured by immunostaining using an antibody corresponding (specifically binding) to the protein, similarly to the case where a protein is selected as a first target substance. Also in cases where a nucleic acid or sugar is selected as a second target substance, the amount of the nucleic acid or sugar is preferably measured by using a nucleic acid probe corresponding (having a base sequence complementary) to the nucleic acid or a lectin that recognizes and binds to the sugar.

Moreover, the amount of the second target substance may be measured by the first quantification method using a fluorescent label such as fluorescent nanoparticle, preferably PID, or by the second quantification method using an enzyme-substrate reaction, such as DAB staining, and is preferably measured by the former method, which has a more excellent quantifying ability and enables drug efficacy evaluation with high accuracy.

The DAB staining method is a technique used in a standard test method to evaluate the expression level of HER2 protein on the cell membrane of, for example, breast cancer cells and then to determine the effectivity of a therapy using an anti-HER2 monoclonal antibody (Trastuzumab) (see HER2 Testing Guideline, Third Edition, Expert Pathologist Panel on Trastuzumab Therapy, September, 2009). The test method evaluates the expression level of HER2 protein in a four-grade scoring system according to the appearance and intensity of staining on the cell membrane of breast cancer cells (staining pattern) as follows: 3+ (positive), more than 30% of cancer cells with intense and complete positive cell membrane staining; 2+ (equivocal), not less than 10% of cancer cells with weak to moderate and complete positive cell membrane staining, or not less than 10% to not more than 30% of cancer cells with intense and complete positive cell membrane staining; 1+ (negative), not less than 10% of cancer cells with faint cell membrane staining which is partial and barely perceptible and is localized only to the cell membrane; and 0 (negative), no positive cell membrane staining, or more than 10% of cancer cells with positive cell membrane staining (cancer cells with positive staining only on cell membranes are excluded from the evaluation). Although such an evaluation method for HER2 expression level may be less quantitative, the evaluation method can be used in the present invention as one embodiment of the quantification method for the amount of the second target substance in the present invention.

The purpose of the method according to one embodiment of the present invention, more specifically the purpose of the amount of a substance, except for human-derived tumor cells, or the index value that reflects the amount of the substance, except for human-derived tumor cells, measured by the method according to one embodiment of the present invention, is not limited to a particular purpose.

The method according to one embodiment of the present invention can be used for quality control of experimental animals, for example, as described below. Two tumor tissue slices are prepared from a tumor region of each experimental animal in a certain generation and one of the slices is used as a sample to prepare sample slides and the other one is transplanted into another immunodeficient animal to prepare an experimental animal in the next generation. The value (or the stratification score) of the measured amount of a first target substance obtained from the above-described sample slides by using the method according to one embodiment of the present invention can be linked to the information on the efficacy of a drug (anti-cancer drug) administered to the above-described experimental animal in the next generation. If the difference in amount of the first target substance in the sample slides is linked to the difference in efficacy of the drug on the experimental animals in the next generation, the amount of the first target substance may potentially affect the quality of experimental animals, which in turns affects the effectivity of a drug. This is also true, for example if there is a tendency that a smaller amount of the first target substance is linked to a higher drug efficacy and a smaller amount of the first target substance is linked to a lower drug efficacy. In that case, experimental animals to be commercialized should be limited to experimental animals having tumor regions containing the first target substance in an amount within a predetermined range that does not affect the effectivity of the drug, or the experimental animals in the subsequent passage.

Such an analysis can also be performed not on the basis of the amount of the first target substance (the total amount in a sample or in the field of view during microscopy) itself, but on the basis of new information derived from the combination of the amount value with other information as described above, such as the amount of the first target substance per one cell.

Furthermore, the purpose of the amount of the substance as a drug efficacy evaluation object owned by the human-derived tumor cells, which is appropriately measured in the method according to one embodiment of the present inventions, or the index value that reflects the amount of the substance is also not limited to a particular purpose.

For example, the analysis of the relationship between the amount of the second target substance and the drug efficacy in the experimental animal in the next generation as well as the above-described relationship between the first target substance and the drug efficacy in the same experimental animal may potentially enable more strict quality, in which, for example, experimental animals to be commercialized are limited to experimental animals having tumor regions containing the first target substance and the amount of the second target substance in amounts within predetermined ranges both of which do not affect the effectivity of a drug, or the experimental animals in the subsequent passage.

### Examples

A preferable aspect of the present invention will be further described below in details by way of Examples, but the present invention is not limited to these Examples.

### [Production Example 1] Production of Texas Red-integrated melamine resin particles

In 22.5 mL of pure water, 2.5 mg of Texas Red (sulforhodamine 101; Sigma-Aldrich Co. LLC.) was dissolved, and the resulting solution was subsequently kept at 70°C with stirring for 20 minutes on a hot stirrer. To the stirred solution, 1.5 g of the melamine resin "NIKALAC MX-035" (Nippon Carbide Industries Co., Inc.) was added, and the resulting solution was further heated with stirring for 5 minutes under the same conditions as above. To the stirred mixture solution, 100 µL of formic acid was added, and the mixture solution was kept at 60°C with stirring for 20 minutes and was then left to cool down to room temperature. The cooled mixture solution was dispensed into centrifuge tubes and then centrifuged at 12,000 rpm for 20 minutes to precipitate Texas Red-integrated melamine resin particles contained in the mixture solution. The supernatant was removed and the precipitated particles were washed with ethanol and water. For the obtained particles, 1000 particles were observed by SEM to measure the average particle diameter and the average particle diameter was determined to be 152 nm.

### [Production Example 2] Production of streptavidin-modified and Texas Red-integrated melamine resin particles

In 1.5 mL of EtOH, 0.1 mg of particles obtained in Production Example 1 was dispersed and 2 µL of the aminopropyltrimethoxysilane "LS-3150" (Shin-Etsu Chemical Co., Ltd.) was added thereto, and the resulting solution was reacted for 8 hours to aminate the surface of the particles.

Then, the above-described surface-aminated particles was adjusted to a concentration of 3 nM by using PBS (phosphate-buffered saline) containing 2 mM EDTA (ethylenediamine tetraacetate), and SM(PEG)₁₂ (Thermo Fisher Scientific Inc., succinimidyl-[(N-maleimidopropionamido)-dodecaethyleneglycol]ester) was added to this solution to a final concentration of 10 mM, and the resulting solution was reacted for 1 hour. This mixture solution was centrifuged at 10,000 g for 20 minutes, and the supernatant was removed, and PBS containing 2 mM EDTA was added to disperse the precipitate, and the resulting mixture was again centrifuged. The same washing step as above was repeated three times to obtain maleimide-modified Texas Red-integrated melamine resin particles.

Meanwhile, streptavidin (Wako Pure Chemical Industries, Ltd.) was treated with N-succinimidyl S-acetylthioacetate (SATA) for thiol addition and then filtered through a gel filtration column to obtain a solution of the resulting streptavidin that was capable of binding to the maleimide-modified and Texas Red-integrated melamine resin particles.

The above-described maleimide-modified Texas Red-integrated melamine resin particles and the thiol-added streptavidin resulting from the above-described treatment were mixed in PBS containing 2 mM EDTA, and the resulting mixture solution was allowed to react at room temperature for 1 hour. Then, 10 mM mercaptoethanol was added thereto to stop the reaction. The obtained mixture solution was concentrated with a centrifugation filter and then purified with a gel filtration column to remove unreacted streptavidin and the like, and consequently streptavidin-modified and Texas Red-integrated melamine resin particles were produced.

### [Example 1]

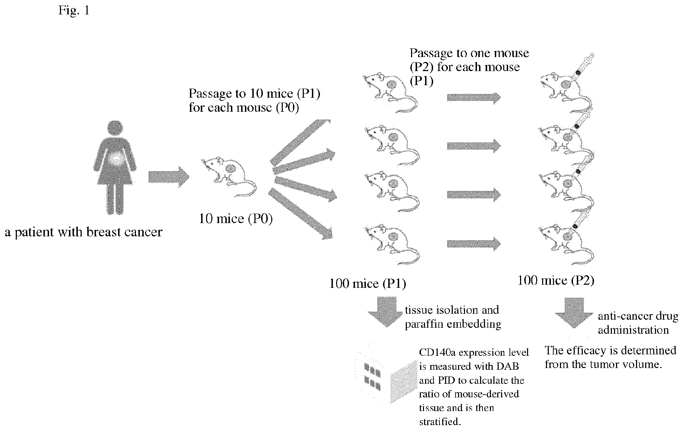

According to the procedures described as below, the expression level of CD140a as a first target substance (protein) in tumor tissue was measured in PDX mice of the first generation and the efficacy of an anti-cancer drug was determined in PDX mice of the second generation. A schematic diagram for Example 1 is shown in Fig. 1.

### [Example 1-1] An embodiment of the measurement for the expression level of CD140a by use of PID

Ten PDX mice (in the 0-th generation: P0) were purchased, which had been prepared by transplanting immunodeficient mice with identical breast cancer tissues (a square of about 3 mm x 3 mm) collected from a patient with breast cancer who had scored +3 in the DAB staining-based HER2 test. Those PDX mice (P0) were grown and measured for tumor size every 3 or 4 days until the tumor volume reached 500 mm³, and then the tumor tissue was removed with a surgical knife from each mouse. The major axis and the minor axis of the tumor region was measured with calipers and the tumor volume was then calculated based on the formula: (major axis) × (minor axis) × (minor axis) × 0.5 (mm³). From the removed tumor tissue, ten square slices of about 3 mm x 3 mm were prepared by using a heat-sterilized surgical or utility knife.

Ten slices each from 10 PDX mice (P0), that is, a total of 100 slices were passaged to other 100 immunodeficient mice to prepare PDX mice (in the first generation: P1). As described above, those PDX mice (P1) were grown and measured for tumor size every 3 or 4 days until the tumor volume reached 500 mm³, and then the tumor tissue was removed with a surgical knife from each mouse. From the removed tumor tissue, two square slices of about 3 mm x 3 mm were prepared by using a heat-sterilized surgical or utility knife.

One out of the two slices was used in the preparation of sample slides to measure the expression level of an antigen called CD140a, which is expressed on the cell surface, such as that of fibroblast, megakaryocyte, monocyte, erythrocyte, bone marrow precursor cells, and endothelial cells. The other one was used in the production of a passage mouse in the second generation (P2).

### <Measurement of CD140a expression level>

### (Sample slide production step)

One out of the two square slices of about 3 mm x 3 mm prepared as described above from the tumor tissue of each PDX mouse (P1) was immersed for 24 hours in 10% neutral buffered formaldehyde (Muto Pure Chemicals Co., Ltd.) for formalin fixation. Then, the fixed tumor tissue was dehydrated by serial treatment with 100% ethanol overnight, 99.5% ethanol for 30 minutes five times, and 100% ethanol for 30 minutes. After dehydration, the tumor tissue was immersed in xylene for 30 minutes twice to replace ethanol in the tumor tissue with xylene. The slice was immersed in melted paraffin for 30 minutes four times in a paraffin wax melter at 60°C for paraffin embedding. The embedded paraffin block was sliced with a microtome for the preparation of sample slides.

### (Immunostaining step)

### • Retrieval

Each sample slide was deparaffinized and then washed for the replacement of the xylene in the tissue with water. The washed sample slide was placed in 10 mM citrate buffer (pH 6.0) and autoclaved at 121°C for 15 minutes to retrieve antigen epitopes. The retrieved sample slide was washed with PBS and the washed sample slide was blocked with PBS containing 1% (w/v) BSA at room temperature for 15 minutes.

### • Antigen-antibody reaction (the primary reaction in the immunostaining of CD140a)

A primary reaction solution containing a rat biotin-conjugated anti-mouse CD140a monoclonal antibody (clone: APA5, BioLegend, Inc.) at a concentration of 5 µg/mL was prepared in PBS containing 1% (w/v) BSA. The sample slide obtained after the retirieval process was immersed in the primary reaction solution and the reaction was allowed to proceed overnight at 4°C.

### • Fluorescence labeling (the secondary reaction)

The streptavidin-modified and Texas Red pigment-integrated melamine resin particle produced in Production Example 2 was diluted to a concentration of 0.02 nM in a diluent containing casein and BSA for fluorescent nanoparticles to prepare a fluorescence labeling solution. The sample slide obtained after the antigen-antibody reaction step was washed in PBS for 5 minutes three times and then immersed in the fluorescence labeling solution, and the reaction was allowed to proceed at room temperature for 3 hours under a neutral pH condition (pH 6.9 to 7.4).

### (Staining step for morphological observation)

The fluorescence-labeled sample slide was stained with Mayer's hematoxylin solution for 5 minutes and then washed with running water at room temperature for 10 minutes.

### (Dehydration, clearing, and mounting steps)

The sample slide obtained after the immunostaining step and the staining step for morphological observation was fixed and dehydrated by immersing the slide in 100% ethanol four times. The sample slide was subsequently cleared with xylene by immersing the slide in xylene four times. The mounting medium "Entellan new" (Merck KGaA) was finally placed between the sample slide and a coverslip to mount the sample and the sample slide for use in the observation (a CD140a/PID-stained sample slide) was consequently obtained.

### (Evaluation process for PID-stained sample slides)

### • Observation and photographing step

In this step, the "BX-53" fluorescence microscope (Olympus Corporation) was used for irradiation with excitation light and for observation of fluorescence emission and the "DP73" digital camera for microscope (Olympus Corporation) mounted on the fluorescence microscope was used to capture immunostaining images (400 times of magnification).

First, the excitation light for Texas Red, which had been used in the fluorescence labeling of CD140a as a first target protein, was irradiated on the specimen for fluorescence emission, and immunostaining images were captured under that condition. In this case, an excitation light filter in the fluorescence microscope was used to select the excitation wavelength of light between 575 and 600 nm and an emission light filter in the fluorescence microscope was used to select the emission wavelength of light between 612 and 692 nm and to observe the emission light that passed through the emission light filter. The intensity of the excitation light in fluorescence microscopy observation and photographing was adjusted such that the irradiance around the image center was 900 W/cm². The exposure time in photographing was controlled to be such a length of time that the brightness would not be saturated in an image, and was set, for example, at 8000 microseconds.

Next, cells stained with hematoxylin for morphological observation were observed and images of those stained cells were captured under bright-field fluorescence microscopy. Such an image of imunostained cells and such an image of differently stained cells for morphological observation were captured within the same field of view, and the same operation was repeated every time the field of view was changed, and images of five different fields of view were captured from one sample slide.

### • Image processing and measurement step

In this step, the "ImageJ" image processing software (open-source software) was used for image processing. The number of bright spots at or above a predetermined brightness on each field of view (screen) was counted and the total brightness of the bright spots was divided by the brightness of one particle of phosphor integrated dot (PID) to calculate a particle number, and the resulting particle number was considered as the expression level of CD140a in the field of view. The expression level of CD140a (particle number) was measured in five different fields of view to calculate the average expression level, which was considered as the "PID score" of the sample slide.

### <Determination of the efficacy of an anti-cancer drug>

Out of the two square slices of about 3 mm x 3 mm prepared as described above from the tumor tissue of each PDX mouse in the first generation (P1), the slice which had not been used in the preparation of sample slides was passaged to another immunodeficient mouse (in the second generation: P2). Repeating this operation for every mouse resulted in the production of 100 PDX mice in the second generation (P2). The slices were measured for tumor volume when they were passaged to these mice. The mice were administered intravenously with the anti-cancer drug Trastuzumab (Product name: Herceptin) at a dose of 15 mg/kg once each on the third day, the fourth day, and the fifth day after the tumor transplantation and were measured for tumor volume three weeks after the administration to calculate relative tumor volume by taking that on the drug administration day as 100. A smaller value of the relative tumor volume means reduction in tumor volume or inhibition of tumor volume growth, from which the drug can be considered to have shown its efficacy.

According to the above-described evaluation process for PID-stained sample slides, the PID score for CD140a on the sample slide was calculated for the 100 PDX mice in the first generation (P1), and the mice were allocated to four groups based on the PID score: P1-1, a PID score of not less than 0 and not more than 50000; P1-2, a PID score of more than 50000 and not more than 100000; P1-3, a PID score of more than 100000 and not more than 150000; and PI-4, a PID score of more than 150000. Moreover, the PDX mice in the second generation (P2) were allocated (linked) to four groups, P2-1, P2-2, P2-3 and P2-4, depending on which of the groups of PDX mice in the first generation (P1), P1-1, P1-2, P1-3 and P1-4, the tumor tissue had been passaged from. The average value of relative tumor volume at three weeks after the above-described drug administration was calculated for each of the groups P2-1, P2-2, P2-3 and P2-4. The drug efficacy in mice with an average tumor volume of 200 or less, more than 200 and not more than 400, more than 400 and not more than 600, and more than 600 was evaluated as excellent (AA), good (BB), fair (CC), and unacceptable (DD), respectively. Table 1 presents the expression level of CD140a measured by using the PID score of each group of PDX mice in the first generation (P1) and the relative value of tumor volume and the determined drug efficacy in the corresponding group of PDX mice in the second generation (see the following section "Analysis").

### [Example 1-2] An embodiment of the measurement for the expression level of CD140a by use of DAB

Similarly to Example 1-1, tumor slices were passaged to 100 PDX mice in the first generation (P1) and two slices of tumor tissue were then produced from each mouse. One out of the two slices was used to prepare CD140a/DAB-stained sample slides by the procedure of Example 1-1 with a modification as described below and then to measure the expression level of CD140a. Moreover, the other slice out of the two was used similarly to Example 1-1 to produce 100 PDX mice in the second generation (P2) passaged with the slice and the efficacy of an anti-cancer drug was further determined in the resulting mice.

For the production of DAB-stained sample slides, the sample slide production step and the immunostaining step including the retrieval and the antigen-antibody reaction were performed similarly to Example 1-1, and the fluorescence labeling was replaced by the DAB staining as described below, and the staining step for morphological observation was not performed, and the dehydration, clearing, and mounting steps were performed similarly to Example 1-1 after the DAB staining.

### (DAB staining)

A solution containing a streptavidin-labeled horseradish peroxidase (SA-HRP, Thermo Fisher Scientific Inc.) at a concentration of 0.1 µg/mL was prepared in PBS containing 1% (w/v) BSA. Each sample slide obtained after the antigen-antibody reaction was immersed in this solution, and the reaction was allowed to proceed at room temperature for 1 hour. Then, the sample slide was washed with PBS for 5 minutes three times and the reaction using a 3, 3'-diaminobenzidine tetrachloride (DAB) solution (Takara Bio Inc.) was subsequently allowed to proceed at room temperature for 10 minutes. The sample slide obtained after the reaction was washed with running water at room temperature for 10 minutes.

### (Evaluation process for DAB-stained sample slides)

### • Observation and photographing step

The Aperio ScanScope CS2 (Leica Biosystems Nussloch GmbH) was used to obtain the DAB-stained area in each sample slide after DAB staining. Specifically, after starting the Aperio ScanScope Console software and placing a sample slide, the sample slide was prescanned by low-resolution imaging of the entire region of the sample. After the prescan, the scan area, the focus point, and the white balance parameters were configured to start image capture. After the image capture, the ImageScope software included in the Aperio was started to select a region for analysis from the list in the annotation window and the Positive Pixel Count (v9) was selected from the analysis algorithms to analyze the DAB-stained region. The positivity (the number of positive pixels identified by DAB staining / the total pixels in a field of view x 100) for CD140a was measured in five fields of view to calculate the average value of positivity, which was considered as the "DAB score" of the sample slide.

### <Determination of the efficacy of an anti-cancer drug>

Similarly to Example 1-1, PDX mice in the second generation (P2) were produced and administered with the anti-cancer drug Trastuzumab and compared for tumor volume between before and after the drug administration to calculate the relative value of tumor volume at three weeks after the drug administration. According to the above-described evaluation process for DAB-stained sample slides, the DAB score for CD140a on the sample slide was calculated for the 100 PDX mice in the first generation (P1), and the mice were allocated to four groups based on the DAB score: PI-5, a DAB score of not less than 0 and not more than 10%; P1-6, a DAB score of more than 10% and not more than 20%; P1-7, a DAB score of more than 20% and not more than 30%; and PI-8, a DAB score of more than 30%. Moreover, the PDX mice in the second generation (P2) were allocated to four groups, P2-5, P2-6, P2-7 and P2-8, depending on which of the groups of PDX mice in the first generation (P1), P1-5, P1-6, P1-7 and P1-8, the tumor tissue had been passaged from. The average value of relative tumor volume at three weeks after the above-described drug administration was calculated for each of the groups P2-5, P2-6, P2-7 and P2-8. The drug efficacy in mice with an average tumor volume of 200 or less, more than 200 and not more than 400, more than 400 and not more than 600, and more than 600 was evaluated as excellent (AA), good (BB), fair (CC), and unacceptable (DD), respectively. Table 2 presents the expression level of CD140a measured by using the DAB staining score of each group of PDX mice in the first generation (P1) and the relative value of tumor volume and the determined drug efficacy in the corresponding group of PDX mice in the second generation.

### <Analysis>

The results from Example 1-1 (Table 1) and Example 1-2 (Table 2) indicated that a group of mice which had been passaged with grafts from mice in the group showing the highest PID or DAB score for CD140a, that is, the group of mice which carried grafts containing a high ratio of mouse-derived stromal cells tended to show an efficacy of the drug which was different from that in mice of the other groups. Thus, the efficacy of a drug in mice can be predicted to some extent by stratifying the expression level of CD140a in the mice. It is understood that this stratification can provide advantages to enable more reproducible experiments and also to save experimental animals from the viewpoint of animal welfare through preliminary exclusion of a group showing the highest PID or DAB score for CD140a from efficacy studies. In this Example, the mice stratified for the expression level of CD140a are one generation different from the mice used for the efficacy study and some alterations in properties should essentially occur between these passages, but a result that could successfully contribute to predicting the drug efficacy to some extent was nevertheless obtained. Therefore, the present invention can be considered to be an effective means to detect alterations in properties even between different generations.

**[Table 1]**

| | Expression level of CD140a^{*1} | Population | | Tumor volume ^{*2} | Drug efficacy^{*3} |
|---|---|---|---|---|---|
| P1-1 | 0-50000 | 59 | P2-1 | 87 | AA |
| P1-2 | 50000-100000 | 26 | P2-2 | 133 | AA |
| P1-3 | 100000-150000 | 9 | P2-3 | 252 | BB |
| P1-4 | >150000 | 6 | P2-4 | 408 | CC |

| | | | | | |
|---|---|---|---|---|---|
| ^{*1}: the number of PID particles in the whole field of view. ^{*2}: the group average value of the tumor volume on Day 21 relative to that when administered (100). ^{*3}: AA, a tumor volume of <200; BB, a tumor volume of 200 to 400; CC, a tumor volume of 400 to 600; DD, a tumor volume of >600. | | | | | |

**[Table 2]**

| | Expression level of CD140a^{*4} | Population | | Tumor volume ^{*2} | Drug efficacy^{*3} |
|---|---|---|---|---|---|
| P1-5 | 0-10% | 61 | P2-5 | 90 | AA |
| P1-6 | 10-20% | 24 | P2-6 | 128 | AA |
| P1-7 | 20-30% | 9 | P2-7 | 252 | BB |
| P1-8 | >30% | 6 | P2-8 | 408 | CC |

| | | | | | |
|---|---|---|---|---|---|
| ^{*2}: the tumor volume on Day 21 relative to that when administered (100). ^{*3}: AA, a tumor volume of <200; BB, a tumor volume of 200 to 400; CC, a tumor volume of 400 to 600; DD, a tumor volume of >600. ^{*4}: analyzed by the Positive Pixel Count (v9) of the Aperio Image Scope. | | | | | |

### [Example 2]

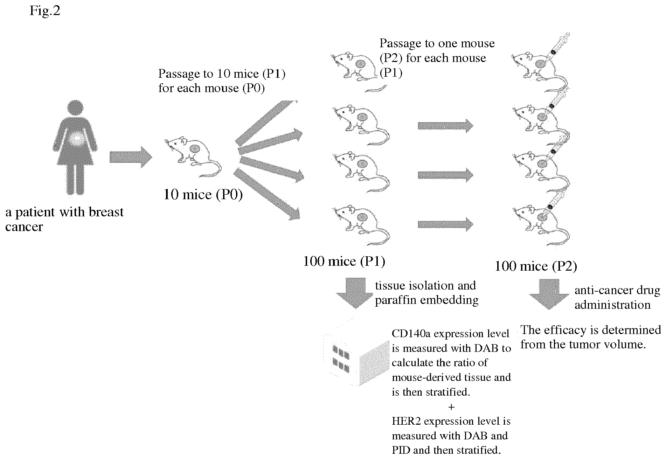

Sample slides prepared from the 100 PDX mice in the first generation (P1) produced in Examples 1-1 and 1-2 (new sample slides that were different from those PID-stained sample slides or DAB-stained sample slides) were used to measure the expression level of HER2 as a second target substance (protein), which is expressed on the cell membrane of, for example, breast cancer cells. A schematic diagram for Example 2 is shown in Fig. 2.

### [Production Example 3] Production of biotin-conjugated anti-rabbit IgG antibody

In a 50 mM Tris solution, 50 µg of an anti-rabbit IgG antibody to be used as a secondary antibody was dissolved. To this solution, a DTT (dithiothreitol) solution was added to a final concentration of 3 mM and the reaction was allowed to proceed at 37°C for 30 minutes. Then, the reaction solution was passed through a desalting column, the "Zeba Desalt Spin Column" (Thermo Fisher Scientific Inc., Cat. #89882), to purify the DTT-reduced secondary antibody. Out of the total volume of the purified antibody, 200 µL of the purified antibody was dissolved in a 50 mM Tris solution to prepare an antibody solution. Meanwhile, the "Maleimide-PEG2-Biotin" linker reagent (Thermo Fisher Scientific Inc., Product Number 21901) was adjusted to a concentration of 0.4 mM with DMSO. This linker reagent solution in a volume of 8.5 µL was added to the antibody solution and mixed, and the reaction was allowed to proceed at 37°C for 30 minutes to conjugate biotin to the anti-rabbit IgG antibody via a PEG chain. This reaction solution was passed through a desalting column for purification. The absorbance of the desalted reaction solution was measured at a wavelength of 300 nm on a spectrophotometer (F-7000; Hitachi High-Tech Science Corporation) to calculate the concentration of the protein (the biotin-conjugated secondary antibody) in the reaction solution. A solution containing the biotin-conjugated secondary antibody at a concentration of 250 µg/mL was prepared in a 50 mM Tris solution and used as a biotin-conjugated secondary antibody solution.

### [Example 2-1] An embodiment of the measurement for the expression level of HER2 by use of DAB

Sample slides prepared by the same method as in the sample slide production step of Example 1-1 were stained by using the VENTANA I-VIEW Pathway HER2 (4B5) kit on the VENTANA BenchMark Ultra system for the assessment of HER2 score on the basis of the DAB method and for the morphological identification of tumor and stromal cell regions. The HER2 scores were in accordance with a commonly used reference standard (see, for example, HER2 Testing Guideline, Third Edition, Expert Pathologist Panel on Trastuzumab Therapy, September, 2009).

### [Example 2-2] An embodiment of the measurement for the expression level of HER2 by use of PID

Each sample slide prepared by the same method as in the sample slide production step of Example 1 - 1 was stained for HER2 contained in the slide by immunostaining using PIDs. The specific procedure of the immunostaining step is as described below.

### (Immunostaining step)

### • Retrieval

Each sample slide prepared by the same method as in the sample slide production step of Example 1 - 1 was deparaffinized and then washed for the replacement of the xylene in the tissue with water. The washed sample slide was placed in 10 mM citrate buffer (pH 6.0) and autoclaved at 121°C for 15 minutes to retrieve antigen epitopes. The retrieved sample slide was washed with PBS and the washed sample slide was blocked with PBS containing 1% BSA at room temperature for 1 hour.

### • Antigen-antibody reaction (the primary reaction in the immunostaining of HER2)

A primary reaction solution containing the "4B5" rabbit anti-HER2 monoclonal antibody (Ventana Medical Systems Inc.) at a concentration of 0.05 nM was prepared in PBS containing 1% (w/v) BSA. The sample slide obtained after the retrieval process was immersed in the primary reaction solution and the reaction was allowed to proceed overnight at 4°C.

### • Secondary antibody reaction (the secondary reaction)

The solution of the biotin-conjugated anti-rabbit IgG antibody produced in Production Example 3 was further diluted to a concentration of 6 µg/mL in PBS containing 1% (w/v) BSA to prepare a secondary reaction solution. The sample slide obtained after the primary reaction was washed with PBS and then immersed in the secondary reaction solution and the reaction was allowed to proceed at room temperature for 30 minutes.

### • Fluorescence labeling (the tertiary reaction)

The streptavidin-modified and Texas Red-integrated melamine resin particle produced in Production Example 1 was diluted to a concentration of 0.02 nM in a diluent for fluorescent nanoparticles containing casein and BSA to prepare a fluorescence labeling solution. The sample slide obtained after the secondary reaction was immersed in the fluorescence labeling solution, and the reaction was allowed to proceed at room temperature for 3 hours under a neutral pH condition (pH 6.9 to 7.4).

### (Staining step for morphological observation)

The fluorescence-labeled sample slide was stained with Mayer's hematoxylin solution for 5 minutes and then washed with running water at 45°C for 3 minutes.

### (Dehydration, clearing, and mounting steps)

The sample slide obtained after the immunostaining step and the staining step for morphological observation was fixed and dehydrated by immersing the slide in 100% ethanol four times. The sample slide was subsequently cleared with xylene by immersing the slide in xylene four times. The mounting medium "Entellan new" (manufactured by Merck KGaA) was finally placed between the sample slide and a coverslip to mount the sample and the sample slide for use in the observation (a HER2/PID-stained sample slide) was consequently obtained.

### (Evaluation process for PID-stained sample slides)

### • Observation and photographing step

In this step, the "BX-53" fluorescence microscope (Olympus Corporation) was used for irradiation with excitation light and for observation of fluorescence emission and the "DP73" digital camera for microscope (Olympus Corporation) mounted on the fluorescence microscope was used to capture immunostaining images (400 times of magnification).

First, the excitation light for Texas Red, which had been used in the fluorescence labeling of HER2 as a second target protein, was irradiated on the sample slide for fluorescence emission, and immunostaining images were captured under that condition. In this case, an excitation light filter in the fluorescence microscope was used to select the excitation wavelength of light between 575 and 600 nm and an emission light filter in the fluorescence microscope was used to select the emission wavelength of light between 612 and 692 nm and to observe the emission light that passed through the emission light filter. The intensity of the excitation light in fluorescence microscopy observation and imaging was adjusted such that the irradiance around the image center was 900 W/cm². The exposure time in photographing was controlled to be such a length of time that the brightness would not be saturated in an image, and was set, for example, at 4000 microseconds.

Next, cells stained with hematoxylin for morphological observation were observed and images of those stained cells were captured under bright-field fluorescence microscopy. Such an image of imunostained cells and such an image of differently stained cells for morphological observation were captured within the same field of view, and the same operation was repeated every time the field of view was changed, and images of five different fields of view were captured from one sample slide.

### • Image processing and measurement step

In this step, the "ImageJ" image processing software (open-source software) was used for image processing. Image processing of the images of staining for morphological observation identified the shapes of cells (the position of the cell membrane) and overlaid those images with the images of immunostaining to extract bright spots representing Texas Red-integrated melamine resin particles that labeled HER2 proteins expressed on the cell membrane. HER2 is not expressed in the stromal cell region. Thus, bright spots inside stromal cells were considered as non-specific signals, namely noise. The number of bright spots at or above a predetermined brightness on the cell membrane was counted and the total brightness of the bright spots was divided by the brightness of one particle of phosphor integrated dot (PID) to calculate a particle number, and the resulting particle number was considered as the expression level of HER2 in the cells. The expression level of HER2 (particle number) was measured in 1000 cells (five different fields of view) per one sample slide to calculate the average expression level, which was considered as the "PID score" of the sample slide.

### <Analysis>

The 100 PDX mice in the first generation (P1) were stratified by the DAB scores in Example 1-2 and further stratified in Example 2-1 by whether the DAB score for HER2 was 3+ or not. Moreover, the results of the anti-tumor study in the PDX mice in the second generation (P2), which had been passaged with grafts from the mice in the first generation, were linked, similarly to Example 1-2, to the DAB scores. The result is presented in Table 3.

Meanwhile, the 100 PDX mice in the first generation (P1) were stratified by the DAB scores in Example 1-2 and further stratified in Example 2-2 by whether the PID score for HER2 was not less than 500 or less than 500. The result is presented in Table 4.

The result shown in Table 3 indicate that stratification of mice by using the scores not only for CD140a but also for HER2 enables selection of PDX mice which are considered to maintain properties suitable for Trastuzumab to show its efficacy, such as the mice in, for example, the groups P2-5-1 and P2-6-1. Furthermore, the result shown in Table 4 indicate that PDX mice which maintain properties suitable for a drug to show its efficacy can be selected by choosing the groups P2-5-3, P2-5-4, P2-6-3 and P2-7-3.

This indicates that mice which express a higher level of HER2 can be further identified by stratification using the PID score among mice evaluated by the DAB score for HER2 as 3+, which in turn indicates the capability of selecting PDX mice which are considered to maintain properties suitable for a drug to show its efficacy at a much higher level.

**[Table 3]**

| | Expression level of CD140a^{*4} | Population | HER2 | Population | | Tumor volume^{*2} | Drug efficacy^{*3} |
|---|---|---|---|---|---|---|---|
| P1-5 | 0-10% | 61 | 3+ | 61 | P2-5-1 | 90 | AA |
| | | | ≤2+ | 0 | P2-5-2 | - | - |
| P1-6 | 10-20% | 24 | 3+ | 23 | P2-6-1 | 118 | AA |
| | | | ≤2+ | 1 | P2-6-2 | 340 | BB |
| P1-7 | 20-30% | 9 | 3+ | 7 | P2-7-1 | 212 | BB |
| | | | ≤2+ | 2 | P2-7-2 | 392 | BB |
| P1-8 | >30% | 6 | 3+ | 5 | P2-8-1 | 358 | CC |
| | | | ≤2+ | 1 | P2-8-2 | 658 | DD |

| | | | | | | | |
|---|---|---|---|---|---|---|---|
| ^{*2}: the tumor volume on Day 21 relative to that when administered(100). ^{*3}: AA, a tumor volume of <200; BB, a tumor volume of 200 to 400; CC, a tumor volume of 400 to 600; DD, a tumor volume of >600. ^{*4}: analyzed by the Positive Pixel Count (v9) of the Aperio Image Scope. | | | | | | | |

**[Table 4]**

| | Expression level of CD140a^{*4} | Population | HER2^{*5} | Population | | Tumor volume^{*2} | Drug efficacy^{*3} |
|---|---|---|---|---|---|---|---|
| P1-5 | 0-10% | 61 | ≥500 | 57 | P2-5-3 | 83 | AA |
| | | | <500 | 4 | P2-5-4 | 192 | AA |

| | Expression level of CD140a^{*4} | Population | HER2^{*5} | Population | | Tumor volume ^{*2} | Drug efficacy^{*3} |
|---|---|---|---|---|---|---|---|
| P1-6 | 10-20% | 24 | ≥500 | 18 | P2-6-3 | 78 | AA |
| | | | <500 | 5 | P2-6-4 | 280 | BB |
| P1-7 | 20-30% | 9 | ≥500 | 6 | P2-7-3 | 120 | AA |
| | | | <500 | 3 | P2-7-4 | 516 | CC |
| P1-8 | >30% | 6 | ≥500 | 3 | P2-8-3 | 207 | BB |
| | | | <500 | 3 | P2-8-4 | 609 | DD |

| | | | | | | | |
|---|---|---|---|---|---|---|---|
| ^{*2}: the tumor volume on Day 21 relative to that when administered (100). ^{*3}: AA, a tumor volume of <200; BB, a tumor volume of 200 to 400; CC, a tumor volume of 400 to 600; DD, a tumor volume of >600. ^{*4}: analyzed by the Positive Pixel Count (v9) of the Aperio Image Scope. ^{*5}: Number of PID particles per one cell | | | | | | | |

## Claims

1. A method comprising the step of measuring an amount of a substance, except for human-derived tumor cells, contained in a sample collected from a region in an experimental animal into which a human-derived tumor tissue or tumor cells have been transplanted, wherein the region contains the tumor tissue or the tumor cells.

2. The method according to claim 1, wherein the substance except for the human-derived tumor cells is stromal cell derived from experimental animal.

3. The method according to claim 1, wherein the substance except for the human-derived tumor cells is stromal cell derived from the human-derived tumor tissue.

4. The method according to claim 2 or 3, wherein the amount of the stromal cells is measured by immunostaining using an antibody against a marker protein that is expressed on the surface of the cells.

5. The method according to any one of claims 1 to 4, further comprising the step of measuring an amount of a substance owned by the human-derived tumor cells contained in the sample.

6. The method according to claim 5, wherein the substance owned by the human-derived tumor cells is a protein, nucleic acid, or sugar, and wherein the protein, nucleic acid, or sugar is quantified by staining using an antibody, nucleic acid probe, or lectin against the protein, nucleic acid, or sugar.

7. The method according to any one of claims 1 to 6, wherein the substance except for the human-derived tumor cells and/or the substance owned by the human-derived tumor cells are/is a protein(s) and the protein(s) is/are measured by a quantifying method using fluorescent nanoparticles.
